(19) Europäisches Patentamt / European Patent Office / Office européen des brevets

(11) **EP 2 338 542 B1**

(12) **FASCICULE DE BREVET EUROPEEN**

(45) Date de publication et mention
de la délivrance du brevet:
**19.08.2015 Bulletin 2015/34**

(51) Int Cl.:
**A61M 1/16** (2006.01)

(21) Numéro de dépôt: **11156262.5**

(22) Date de dépôt: **17.06.2004**

(54) **Dispositif de traitement de sang par circulation extracorporelle**

Extrakorporelle Blutbehandlungsvorrichtung mit automatischer Entleerung der gebrauchten Flüssigkeit

Extracorporal device for treating blood with automatic exhaust of the used fluid

(84) Etats contractants désignés:
**AT BE BG CH CY CZ DE DK EE ES FI FR GB GR HU IE IT LI LU MC NL PL PT RO SE SI SK TR**

(30) Priorité: **25.06.2003 FR 0307643**

(43) Date de publication de la demande:
**29.06.2011 Bulletin 2011/26**

(62) Numéro(s) de document de la (des) demande(s)
initiale(s) en application de l'article 76 CBE:
**04743770.2 / 1 635 895**

(73) Titulaire: **Gambro Lundia AB**
**220 10 Lund (SE)**

(72) Inventeur: **Chevallet, Jacques**
**FR-69360, Serezin du Rhone (FR)**

(74) Mandataire: **Ponzellini, Gianmarco et al**
**PGA S.r.l.**
**Via Mascheroni, 31**
**20145 Milano (IT)**

(56) Documents cités:
**EP-A- 0 796 997     JP-A- 53 083 397**
**JP-A- 59 037 959     US-A- 4 267 041**
**US-A- 4 859 319     US-A- 5 043 074**

**Description**

Domaine technique de l'invention

La présente invention concerne un dispositif de traitement extracorporel du sang, et plus particulièrement, un appareil innovant et amélioré pour le traitement du sang dans lequel on peut effectuer une vidange du liquide usé de façon automatique.

Etat de la technique antérieure :

Le traitement extracorporel du sang implique de prélever du sang d'un patient, de traiter le sang extérieurement au patient, de renvoyer le sang traité au patient. Le traitement extracorporel de sang est utilisé typiquement pour extraire des matières ou des molécules indésirables du sang du patient, et/ou pour ajouter des matières ou des molécules bénéfiques au sang. Le traitement extracorporel de sang est utilisé avec des patients incapables d'éliminer efficacement des matières de leur sang, par exemple dans le cas d'un patient qui souffre d'une défaillance temporaire ou permanente des reins. Ces patients et d'autres patients peuvent suivre un traitement extracorporel du sang pour ajouter ou éliminer des matières dans leur sang, pour maintenir un équilibre acide-base ou pour éliminer des fluides corporels excessifs, par exemple.

Le traitement extracorporel du sang est typiquement effectué en prélevant le sang du patient en un flux continu, en introduisant le sang dans une chambre primaire d'un filtre dans laquelle le sang passe à travers une membrane semi - perméable. La membrane semi-perméable laisse passer, de manière sélective, des matières indésirables contenues dans le sang à travers la membrane, de la chambre primaire vers la chambre secondaire, et laisse également passer, de manière sélective, des matières bénéfiques contenues dans le liquide passant dans la chambre secondaire à travers la membrane vers le sang passant dans la chambre primaire, en fonction du type de traitement.

Un certain nombre de traitement extracorporel de sang peuvent être effectués par une même machine. Dans un traitement par ultrafiltration (UF), les matières indésirables sont éliminées du sang par convection à travers la membrane dans la chambre secondaire.

Dans un traitement par hémofiltration (HF), le sang s'écoule à travers la membrane semi-perméable comme dans l'UF, et des matières bénéfiques sont ajoutées au sang, typiquement par introduction d'un fluide dans le sang, soit avant, soit après son passage à travers le filtre et avant qu'il ne soit renvoyé au patient.

Dans un traitement par hémodialyse (HD), un fluide secondaire contenant des matières bénéfiques est introduit dans la chambre secondaire du filtre. Les matières indésirables du sang traversent la membrane semi-perméable et pénètrent dans le fluide secondaire et les matières bénéfiques du fluide secondaire peuvent traverser la membrane et pénétrer dans le sang.

Dans un traitement par hémodiafiltration (HDF), le sang et le fluide secondaire échangent leurs matières comme dans l'HD, et en outre, des matières sont ajoutées au sang, typiquement en introduisant un fluide dans le sang traité avant qu'il ne soit renvoyé au patient comme dans l'HF, et les matières indésirables sont aussi éliminées du sang par convection.

Dans chaque traitement, le fluide secondaire passe à travers la chambre secondaire du filtre et reçoit les matières indésirables du sang par le biais de la membrane. Ce liquide est ensuite extrait du filtre : il est communément appelé liquide usé, et est emmené vers un égout ou vers un recueil destiné ensuite à être vidé dans un égout.

Comme il a été décrit précédemment, un patient peut souffrir d'une défaillance temporaire ou d'une défaillance permanente des reins.

Dans le cas d'une défaillance permanente des reins, le patient devra subir d es séances régulières, par exemple trois fois par semaine, de traitement extracorporel sanguin avec un débit d'extraction de sang relativement élevé, à savoir entre 200 et 500mL/min. Généralement, le personnel soignant peut installer le dispositif de traitement avec une préparation en ligne de liquide pouvant comporter une stérilisation en ligne, et peut installer un égout en ligne.

Dans le domaine d'une défaillance temporaire des reins, le patient devra être traité en urgence et devra subir un traitement extracorporel sanguin continu et de longue durée avec un débit d'extraction de sang relativement faible, c'est-à-dire entre 100 et 200mL/min. L'extraction nette d'eau du patient est limitée car le patient traité en urgence est dans un état critique.

Dans ce cas de traitement en urgence, le personnel soignant doit agir rapidement et n'a donc pas le temps d'installer l'appareil de traitement avec une préparation en ligne de liquide. Il est en effet beaucoup plus rapide d'accrocher au dispositif un liquide de dialyse et/ou un liquide d'infusion déjà préparé et stocké dans un sac jetable stérile, et il est plus rapide d'accrocher un sac jetable vide pour recueillir le liquide usé.

Une machine adoptant cette solution de sacs stériles jetables est connue. Pendant la séance de traitement intensif de l'insuffisance rénale, cette machine de traitement extracorporel doit assurer et contrôler plusieurs débits :

- le débit de perfusion (« infusion » en anglais) (Dinf), dans le cas où une infusion de liquide avec des matières bénéfiques est prescrite au patient,
- le débit de liquide de dialyse (Ddial) entrant dans la chambre secondaire du filtre dans le cas d'un mode HF ou HDF,
- le débit représentant la perte de poids (weight loss) par le patient (Dwloss), c'est-à-dire la quantité de liquide extraite et perdue par le patient,
- le débit représentant le liquide usé (waste) sortant du filtre Dwaste.

Le système représenté par le patient et l'appareil de traitement sanguin est un système fermé. Ainsi on peut en déduire l'équation suivante :

$$\text{Dwaste} = \text{Dinf} + \text{Ddial} + \text{Dwloss} \quad (1)$$

Aussi, avant la séance de traitement, le médecin peut prescrire :

- le débit de perfusion Dinf pour contrôler la quantité de matières bénéfiques à perfuser au patient,
- le débit de dialyse Ddial pour contrôler le passage de matières à travers le filtre,
- le débit de perte de poids Dwloss par le patient pour éviter tout malaise potentiel du patient pendant la séance.

Par conséquent le débit de liquide usé est calculé selon l'équation (1).

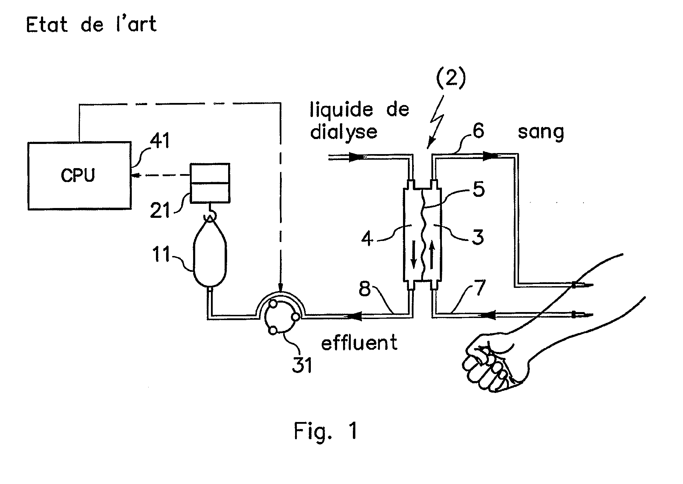

Pour cela, il a été décrit plus haut l'utilisation d'un sac jetable stérile permettant de recevoir et collecter le liquide usé. Cette utilisation connue est illustrée en figure 1. Le sac 11 est connecté à l'extrémité de la ligne 8 de liquide usé reliée à la chambre secondaire 4. Ce sac 11 est associé à un moyen de pesage gravimétrique 21 relié à une unité de contrôle 41. Ainsi, des signaux de poids sont transmis à l'unité de contrôle 41 qui est capable de suivre l'évolution du poids du sac liée au débit de liquide usé à travers la ligne 8 de liquide usé, et de commander une pompe 31 active sur la ligne de liquide usé.

Cependant, la séance peut durer plusieurs jours et le sac jetable de liquide usé se trouve rempli bien avant la fin de la séance. Ce phénomène est d'autant plus constaté lors d'un traitement intensif. En effet, on souhaite d'une part échanger une quantité de liquide élevée en thérapie HF ou HDF, et d'autre part on veut effectuer des traitements de longue durée.

Dès que le sac atteint un certain niveau de remplissage, le médecin ou l'infirmière intervient sur la machine pour arrêter temporairement les pompes respectivement actives sur la ligne de liquide usé, sur la ligne de liquide de dialyse et sur la ligne d'infusion, tandis que le sang continue à circuler extracorporellement dans la chambre primaire du filtre. Une fois les pompes arrêtées, l'utilisateur doit débrancher et décrocher le sac rempli de liquide usé, le vider et/ou le jeter dans un réseau de vidange. Puis l'utilisateur accroche et branche un nouveau sac vide à usage unique sur le dispositif de traitement et remet en fonctionnement les pompes pour revenir au traitement extracorporel avec circulation des fluides à travers les deux chambres (3, 4) du filtre 2.

Cette opération de remplacement de sac présente des inconvénients :

- d'une part, elle peut durer quelques minutes et rallonge le temps de traitement de quelques minutes à chaque fois que le sac est rempli et nécessite d'être changé,
- d'autre part, cette opération de changement de sac s'effectue alors que le sang circule toujours dans le circuit sanguin sans pouvoir être en contact avec un liquide de dialyse passant, la qualité du traitement est dès lors moindre,
- aussi, cette opération est effectuée par du personnel soignant qui doit souvent suivre plusieurs patients en même temps. Un temps d'attente avant l'intervention du personnel peut encore s'ajouter au temps de traitement,
- en outre, le changement régulier du sac de vidange pendant une séance rajoute un coût économique au traitement,
- enfin, les sacs sont généralement d'un volume d'environ cinq litres, représentent un objet lourd et relativement fragile à manipuler et contiennent un liquide usé pouvant représenter une source de substances indésirables si le sac était malencontreusement perforé pendant la manipulation.

Le document EP0796997 est relative à un dispositif de dialyse. Le dispositif comprends un filtre ayant une chambre primaire et une chambre secondaire séparées par une membrane semi-perméable, un circuit sang comportant une ligne artérielle destinée à sortir du patient, la chambre primaire du filtre et une ligne veineuse destiné à retourner au patient, un circuit dialysat comportant la chambre secondaire du filtre et une ligne de vidange et un premier et un second récipient **reliés** à un tube d'évacuation du liquide usé. Un première et un deuxième balance mesure le poids du liquide dans le premier et un second récipient.

Le document US 4859319 et le document US 50430745 concernent un dispositif de mesure de la quantité d'ultrafiltration éliminée pendant un traitement de dialyse. Le dispositif comprends un premier et un second récipient reliés à un tube d'évacuation et des sondes de mesure capable de détecter la présence d'ultrafiltrat à un niveau prédéterminé dans le premier et le second récipient et des moyens d'élaboration capables d'établir la quantité d'ultrafiltrat éliminé.

Exposé de l'invention:

La présente invention est décrite en référence particulière au traitement intensif de l'insuffisance rénale (encore appelé traitement de l'insuffisance rénale aiguë), sans par là limiter la portée de l'invention à cette application spécifique.

L'invention a pour objet un dispositif de traitement de sang selon la revendication 1.

Brève description des dessins :

Des caractéristiques et avantages supplémentaires apparaîtront à la description détaillée d'un mode de réalisation préféré mais non exclusif d'un dispositif de traitement extracorporel sanguin selon l'invention. Cette description sera effectuée ci-dessous en référence aux dessins annexés, qui sont fournis à titre indicatif et donc non limitatif.

Il est décrit quatre modes de réalisation de l'invention.

La figure 1 représente un dispositif de traitement extracorporel de sang selon l'état connu de la technique.

La figure 2 représente un premier mode de réalisation du dispositif selon l'invention.

La figure 3 représente la première phase de fonctionnement du premier mode de réalisation du dispositif selon l'invention.

La figure 4 représente la seconde phase de fonctionnement du premier mode de réalisation du dispositif selon l'invention.

La figure 5 représente l'évolution du poids mesuré de sacs pendant la séance de traitement du premier mode de réalisation.

La figure 6 représente un deuxième mode de réalisation du dispositif selon l'invention.

La figure 7 représente la première phase de fonctionnement du deuxième mode de réalisation du dispositif selon l'invention.

La figure 8 représente la seconde phase de fonctionnement du deuxième mode de réalisation du dispositif selon l'invention.

La figure 9 représente un troisième mode de réalisation du dispositif selon l'invention.

La figure 10 représente la première phase de fonctionnement du troisième mode de réalisation du dispositif selon l'invention.

La figure 11 représente la seconde phase de fonctionnement du troisième mode de réalisation du dispositif selon l'invention.

La figure 12 représente un quatrième mode de réalisation du dispositif selon l'invention.

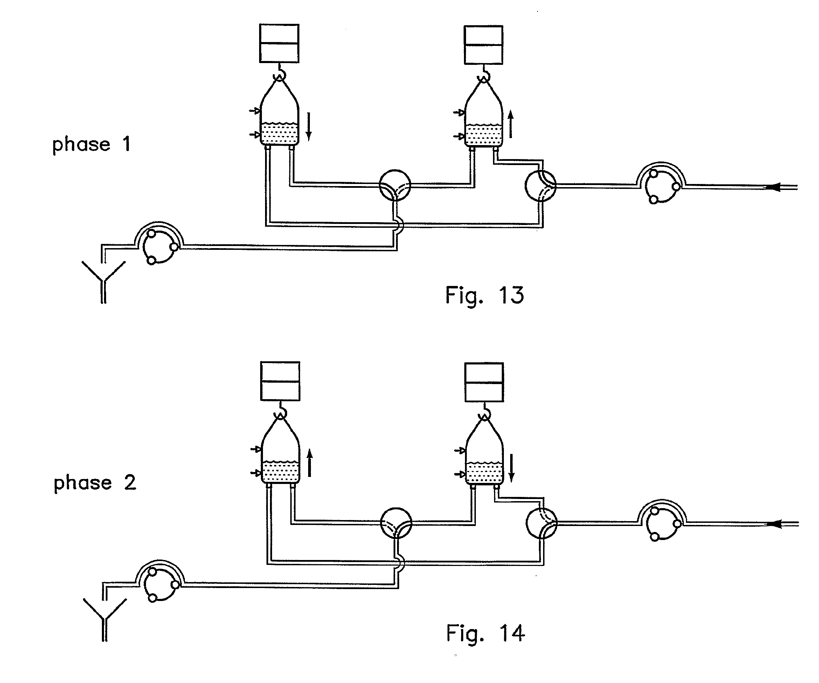

La figure 13 représente la première phase de fonctionnement du quatrième mode de réalisation du dispositif selon l'invention.

La figure 14 représente la seconde phase de fonctionnement du quatrième mode de réalisation du dispositif selon l'invention.

Exposé détaillé de modes de réalisation de l'invention :

Description commune à tout mode :

En référence aux figures annexées, on a désigné globalement par 1 le dispositif de traitement extracorporel sanguin. Le dispositif de traitement de sang 1 est représenté dans les figures 1, 2, 6, 9 et 12 est dans une configuration fonctionnelle qui lui permet d'effectuer un traitement d'hémodialyse. Les autres configurations de traitement citées plus haut (ultrafiltration, hémofiltration et hémodiafiltration) sont bien entendu transposables aux modes de réalisation de l'invention.

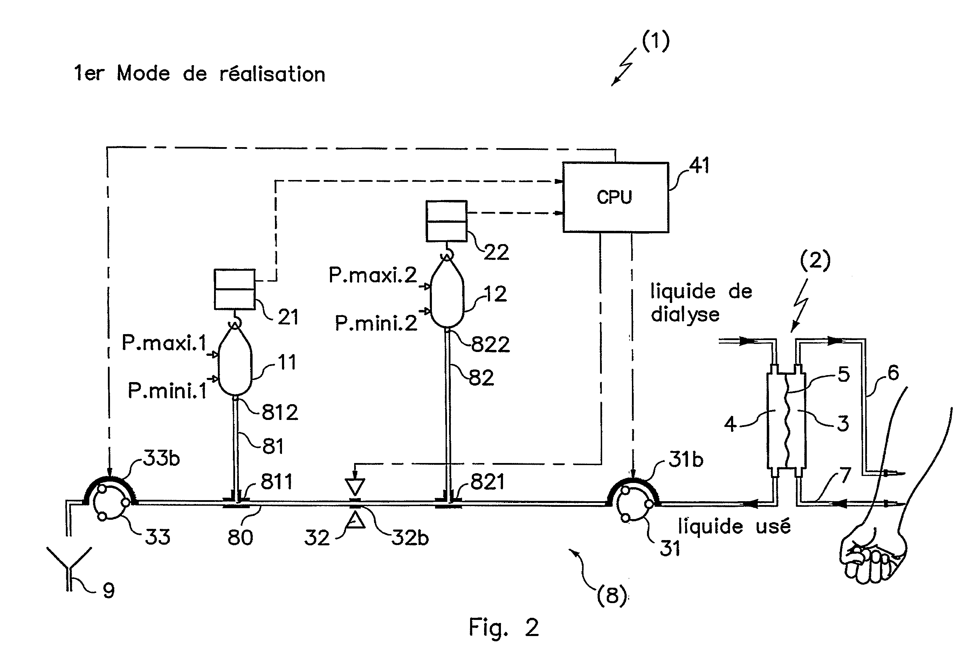

Les dispositifs selon les différents modes de réalisation de l'invention représenté dans les figures 2, 6, 9, 12 comportent un filtre 2 ayant une chambre primaire 3 et une chambre secondaire 4 séparées par une membrane semi-perméable 5 ; un circuit sang comporte une ligne artérielle 6 destinée à sortir du patient, la chambre primaire 3 du filtre et une ligne veineuse 7 destinée à retourner au patient ; un circuit dialysat comporte la chambre secondaire 4 du filtre et au moins une ligne de vidange 8 pour la circulation du liquide usé destiné à sortir du filtre 2 et destiné à aller vers un égout 9, un premier sac 11 en communication de fluide avec la ligne de vidange 8, au moins un premier moyen de pesage gravimétrique 21 associé au premier sac 11, des moyens de réglage de débit de fluide (31, 32, 33, 34) actifs sur la ligne de vidange 8 ; une unité de contrôle 41 est reliée au premier moyen de pesage gravimétrique 21 et aux moyens de réglage de débit de fluide (31, 32, 33, 34). Les dispositifs selon les différents modes de réalisation de l'invention comportent un second sac 12 en communication de fluide avec la ligne de vidange 8.

L'unité de contrôle 41 est capable de recevoir les signaux de poids du premier moyen de pesage gravimétrique et de commander les moyens de réglage de débit de fluide (31,32,33,34) pour charger de liquide un des sacs (11, 12) pendant que l'autre sac (12, 11) se décharge de liquide, et vice-versa.

Dans tout mode de réalisation, l'unité de contrôle 41 est également capable de calculer, à partir des signaux de poids reçus, la quantité de liquide sortant du filtre et entrant dans la ligne de vidange 8.

Plus particulièrement et dans tout mode de réalisation, les moyens de réglage comportent un premier organe de réglage 31 actif en amont des deux sacs (11, 12). Le premier organe est donc actif sur la ligne de vidange, après la sortie de la chambre secondaire du filtre, en amont des sacs. Aussi l'unité de contrôle 41 sera programmée pour commander le premier organe d e réglage 31 afin de garantir la présence d'un débit substantiellement continu pendant le traitement. En effet, les moyens de réglage de débit sont commandés de sorte à ne plus devoir arrêter le flux de liquide usé sortant du filtre. Pour une qualité meilleure du traitement, on veillera à ce que le débit de liquide usé mesuré reste substantiellement constant ou suive un profil souhaité pendant la séance.

Dans tout mode de réalisation, les moyens de réglage peuvent comporter un deuxième organe de réglage 32 actif entre les deux sacs (11, 12).

Dans tout mode de réalisation, les moyens de réglage peuvent comporter un troisième organe de réglage 33 actif en aval des deux sacs (11, 12).

Des caractéristiques additionnelles sont possibles pour tout mode de réalisation.

Ainsi, le dispositif peut comporter un second moyen de pesage gravimétrique 22 associé au second sac 12 et relié à l'unité de contrôle 41. En effet, l'information de poids fournie à l'unité de contrôle par le premier moyen de pesage gravimétrique a pour fonction de connaître le poids du sac à usage unique à la fois pour savoir la quantité de liquide passant, et pour commander la phase de charge et décharge des deux sacs en utilisant deux valeurs seuils maximale et minimale prédéterminées par l'utilisateur en fonction de la contenance du sac.

L'association d'un second moyen de pesage gravimétrique au second sac est possible. Le second moyen de pesage gravimétrique a pour première fonction de connaître le poids du liquide passant. Il peut également servir au contrôle du processus cyclique de charge et décharge en utilisant une ou deux valeurs seuil du second moyen de pesage gravimétrique, en association avec une ou deux valeurs seuils du premier moyen de pesage gravimétrique. Mais si l'utilisation des deux valeurs seuils suffit pour le contrôle de la vidange, les quatre valeurs seuil des deux pesons peuvent être utilisées dans un but préventif d'alarme concernant l'état anormal d'un sac.

Dans tout mode de réalisation, l'unité de contrôle 41 est capable de calculer la quantité de fluide sortant du filtre 2 et entrant dans la ligne de vidange 8 à partir des signaux reçus du premier moyen de pesage gravimétrique 21 et/ou du second moyen de pesage gravimétrique 22.

Pour tout mode de réalisation, l'unité de contrôle 41 est capable d'activer une procédure de contrôle comportant deux phases alternées.

Dans une première phase, l'unité de contrôle commande le débit réel des moyens de réglage (31, 32, 33, 34) comme fonction du profil de débit souhaité et de l'information de poids venant d'au moins le premier moyen de pesage gravimétrique 21.

Dans une seconde phase, l'unité de contrôle 41 commande le débit réel des moyens de réglage (31, 32, 33, 34) comme fonction du profil de débit souhaité et de l'information de poids venant des premier et second moyens de pesage gravimétrique (21,22).

L'unité de contrôle 41 est également capable d'activer une procédure de contrôle comportant deux phases alternées avec un contrôle de débit réel des moyens de réglage (31, 32, 33, 34) lors de la première phase qui s'effectue également en fonction de l'information de poids du second moyen de pesage gravimétrique 22. Cette alternative peut être employée dans les troisième et quatrième modes de réalisation.

Dans tout mode de réalisation, l'unité de contrôle 41 est capable de recevoir l'information de poids du premier moyen de pesage gravimétrique 21 et/ou du second moyen de pesage gravimétrique 22, calculer le débit réel de fluide sortant du filtre 2 et le comparer à un débit souhaité prédéterminé ou à un profil de débit souhaité par l'utilisateur, contrôler le débit réel de fluide par les moyens de réglage pour approcher au meilleur le profil de débit souhaité de fluide sortant du filtre 2.

Dans tout mode de réalisation, l'unité de contrôle est capable de recevoir l'information de poids du premier moyen de pesage gravimétrique 21 et/ou du second moyen de pesage gravimétrique 22, déterminer indépendamment l'état de remplissage de chaque sac, commander, à partir de l'état de remplissage de chaque sac, une procédure de charge et décharge alternée et successive des sacs.

Dans tout mode de réalisation, l'unité de contrôle est capable de recevoir l'information de poids du premier moyen de pesage gravimétrique 21 et/ou du second moyen de pesage gravimétrique 22, détecter des valeurs de seuil maximal et seuil minimal pour chacun des sacs $P_{1mini}$, $P_{1maxi}$, $P_{2mini}$, $P_{2maxi}$, commander à partir de valeurs de seuil une procédure de charge et décharge des sacs selon les étapes suivantes :

- o charge d'un sac et décharge de l'autre sac,
- o détection d'un seuil limite,
- o décharge d'un sac et charge de l'autre sac,
- o détection d'un autre seuil limite.

Description commune au premier et deuxième modes de réalisation :

Dans les deux premiers modes de réalisation de l'invention, la ligne de vidange 8 peut être définie avec plusieurs composantes : un conduit 80 destiné à relier le filtre 2 à l'égout 9, un premier branchement 81 reliant le premier sac 11 au conduit 80, un deuxième branchement 82 reliant le second sac 12 au conduit 80. Le deuxième branchement 82 est connecté au conduit 80 en amont du premier branchement 81. Aussi le premier organe de réglage 31 et le deuxième organe de réglage 32 sont actifs sur le conduit 80, et non sur les deux branchements.

Chacun des deux branchements peut comporter une ligne avec deux raccords terminaux respectifs (811, 812, 821, 822), ou bien un raccord direct entre la ligne de vidange (80) et une ouverture d'un sac.

Description du premier mode de réalisation :

Dans le premier mode de réalisation illustré en figure 2, les premier 31 et troisième 33 organes de réglage sont opportunément des pompes péristaltiques, et le deuxième organe de réglage 32 est une vanne.

Dans le premier mode de réalisation illustré en figure 2, il faut tenir compte de la place dans l'espace des sacs. Il est connu que le ou les sacs jetables utilisés sont accrochés à la machine avec l'ouverture des sacs placée opportunément vers le bas pour permettre un écoulement du fluide continu. On peut trouver plusieurs sacs notamment un sac jetable collectant le liquide usé, un sac jetable contenant le liquide de perfusion, un sac jetable comportant un liquide de dialyse. Ces sacs sont souvent accrochés au même niveau.

L'invention utilise la gravité pour faciliter l'écoulement de liquide sans forcément recourir à une pompe supplémentaire. Cette utilisation est faite dans le premier mode de réalisation, mais la gravité pourrait être utilisée dans les autres modes par l'homme du métier à l'aide de ses connaissances et l'exposé de l'invention.

Ainsi, le premier sac 11 est placé plus bas que le second sac 12 sur la machine. Par conséquent, lorsqu'un liquide usé est amené depuis la chambre secondaire du filtre et qu'un passage de fluide est possible entre les deux sacs, le premier sac 11 se chargera en priorité par rapport au second sac, même si le second sac est placé en amont du premier sac dans le sens de circulation du fluide. De la même façon, lorsque le second sac sera rempli et le deuxième substantiellement vide, le second sac 12 se déchargera dans le premier sac 11 par gravité.

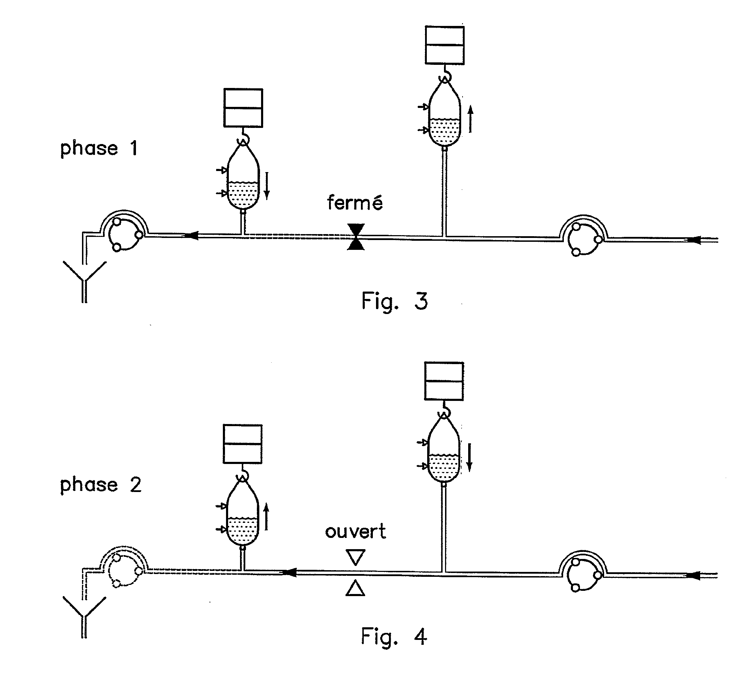

Les figures 3 et 4 illustrent les deux phases de fonctionnement du cycle de vidange instauré par l'appareil et les sens de passage de fluide dans la ligne de vidange, pour le premier mode de réalisation.

En effet, l'unité de contrôle 41 est capable de commander les organes de réglage (31, 32, 33) selon deux phases alternées.

En première phase, l'unité de contrôle 41 commande l'ouverture du deuxième organe de réglage 32 et l'arrêt du troisième organe de réglage 33 pour la charge du second sac 12 et la décharge du premier sac 11 dans l'égout 9.

En seconde phase, l'unité de contrôle 41 commande l'ouverture du premier organe de réglage 31 et l'arrêt du troisième organe de réglage 33 pour la décharge du second sac 12 et la charge du premier sac 11.

On notera que la commande pour passer d'une phase à l'autre doit s'effectuer de manière opportune de façon simultanée afin d'avoir une meilleure qualité de traitement, mais un petit intervalle de temps pourra être constaté entre deux actionnements, par exemple entre l'ouverture du deuxième organe de réglage 32 et l'arrêt du troisième organe de réglage 33. Ceci est valable pour toute commande de tout moyen de réglage.

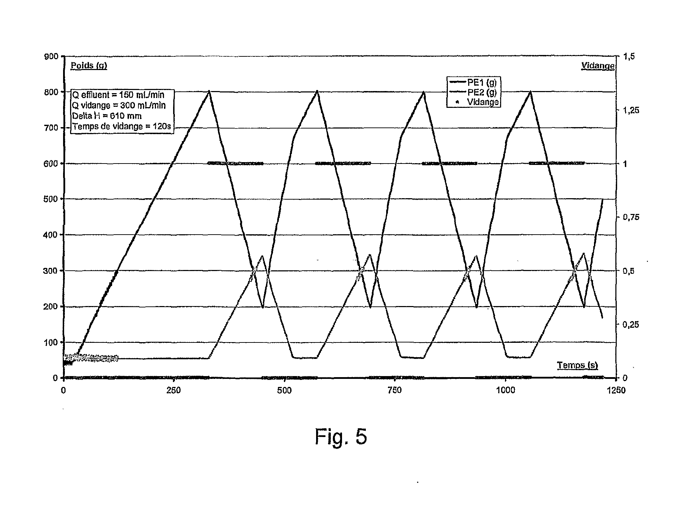

La figure 5 représente, pour le premier mode de réalisation, l'évolution du poids de chaque sac en fonction du temps de traitement. Ces mesures ont été effectuées expérimentalement et sont reproductibles.

On va maintenant expliquer, à partir du cas particulier de la figure 5, la succession des deux phases pendant le cycle de vidange, précédée d'une phase d'amorçage du système. "

Au début de la séance, les deux sacs sont presque vides (un poids de 50g est enregistré), une phase d'amorçage est mise en oeuvre.

L'unité de contrôle amorce le premier moyen de réglage 31, ouvre le deuxième organe de réglage 32, et ne fait pas fonctionner le troisième moyen de réglage 33.

Le premier organe de réglage commande le passage du liquide usé dans la ligne de vidange 8. Dès lors, le liquide va passer dans le conduit 80.

Or le premier sac 11 est en aval par rapport au second sac 12, mais est accroché au dispositif plus bas que le second sac. Plus particulièrement, la limite haute du premier sac est placée plus bas ou au même niveau que la limite basse du second sac. Le second sac se charge en priorité par rapport au premier sac.

Ainsi, on constate que le poids du premier sac 11 augmente régulièrement en priorité par rapport au poids du second sac 12 qui reste inchangé.

Dès que le premier sac 11 atteint un poids maximal prédéterminé $P_{1maxi}$ (800g pour l'essai), le dispositif va fonctionner selon une première phase: le premier organe de réglage 31 continue à fonctionner, le second organe de réglage 32 est fermé et le troisième organe de réglage fonctionne pour conduire le liquide vers l'égout.

Dès lors le premier sac 11 dont le poids aura été mémorisé par l'unité de contrôle, va se décharger dans l'égout. On constate que le poids du premier sac diminue régulièrement de 800g à 200g.

D'autre part, le second sac se charge avec le liquide usé sortant du filtre. On constate une augmentation du poids du second sac 22 de 50g à 330g environ.

Cette phase s'effectuera jusqu'à ce que, soit un seuil minimal de poids du premier sac $P_{1mini}$ est atteint (200g), soit un seuil maximal de poids du second sac $P_{2maxi}$ (330g) est atteint, soit le premier des deux seuils précités est atteint.

A la détection d'un tel seuil, l'unité de contrôle commande l'entrée en seconde phase.

L'unité 41 commande l'ouverture du deuxième organe de réglage 32 et l'arrêt du fonctionnement du troisième organe de réglage 33. Ainsi, le second sac presque rempli, dont l'information de poids pourra être mémorisée par l'unité de contrôle, se décharge dans le premier sac presque vide. On constate que le premier sac se remplit non seulement avec le liquide contenu dans le second sac 22 mais aussi avec le liquide sortant directement du filtre. C'est pourquoi on constate une inflexion de la droite représentant l'augmentation régulière de poids pendant la seconde phase : le second sac est presque vidé à cet instant et la charge du premier sac s'effectuera moins rapidement (50g).

Ainsi vont s'alterner première et seconde phase jusqu'à la fin de la séance.

La taille de chacun des sacs, la taille des lignes jetables sont prédéterminées par l'utilisateur avant la séance. Dans l'essai conduit avec le premier mode de réalisation, le second sac a une contenance de 500g environ alors que le premier sac a une plus grande contenance, d'environ 1 kg, les lignes ont la même taille. Les débits adoptés pendant la séance s'accordent bien entendu à la taille des sacs et ligne et sont tels que le premier sac 11 atteint un poids minimal prédéterminé avant que le second sac 12 n'atteigne un poids maximal prédéterminé. Dans l'essai illustré, le débit de vidange est de 300mL/min et le débit au travers du premier organe réglage est de 150mL/min.

Description du deuxième mode de réalisation:

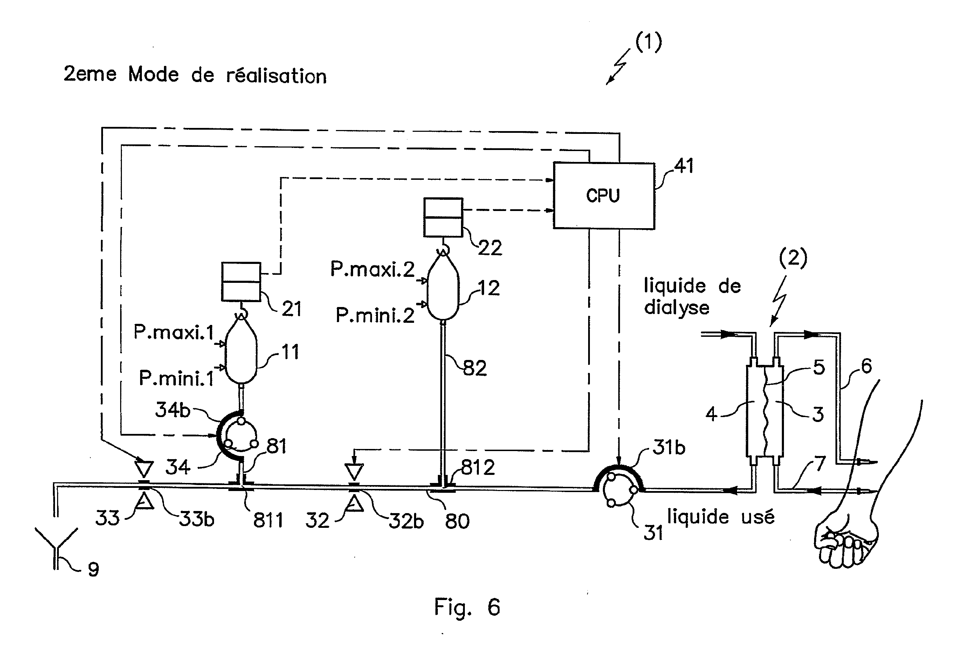

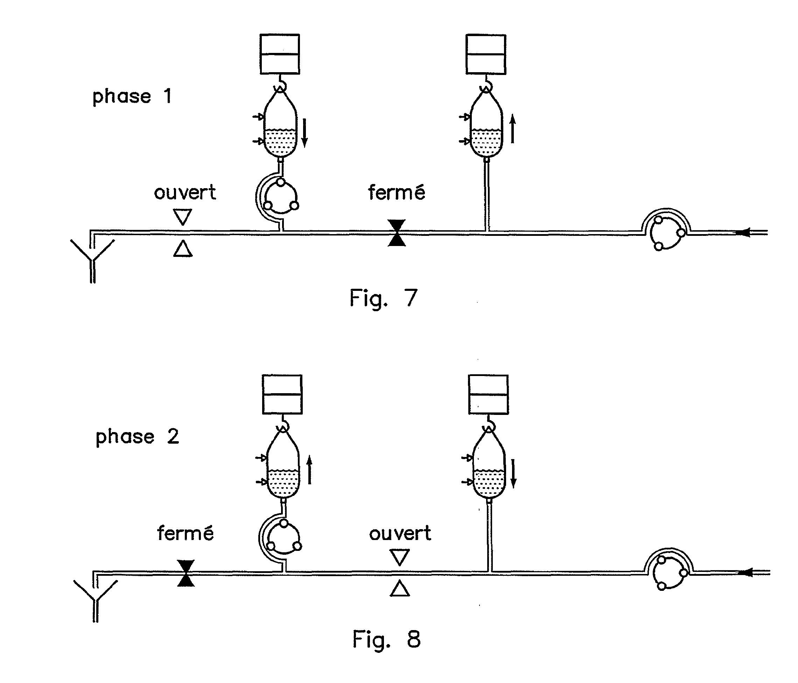

Un deuxième mode de réalisation est illustré en figure 6 et les deux phases de fonctionnement sont représentées en figure 7 et 8.

Dans le deuxième monde dé réalisation, les moyens de réglage comportent un quatrième organe de réglage 34 actif sur le premier branchement 81 entre les deux raccords (811, 812).

Ce quatrième organe de réglage 34 peut comporter indifféremment une pompe, plus particulièrement une pompe péristaltique, une vanne, plus particulièrement un clamp deux voies ou une vanne à ouverture réglable.

Plus particulièrement dans le deuxième mode de réalisation, le premier et le quatrième organes de réglage (31, 34) peuvent être des pompes péristaltiques et les deuxième et troisième organes de réglage (32,33) peuvent être des vannes.

Dans le deuxième mode de réalisation, l'unité de contrôle 41 est capable de commander les moyens de réglage de débit (31, 32, 33, 34) selon deux phases alternées.

En première phase, l'unité de contrôle 41 commande la fermeture du deuxième organe de réglage 32, l'ouverture du troisième organe de réglage 33, l'actionnement du quatrième organe de réglage 34 dans le sens sac-conduit,

En seconde phase, l'unité de contrôle 41 commande l'ouverture du deuxième organe de réglage 32, la fermeture du troisième organe de réglage 33, l'actionnement du quatrième organe de réglage 34 dans le sens conduit-sac.

Description commune aux troisième et quatrième modes de réalisation :

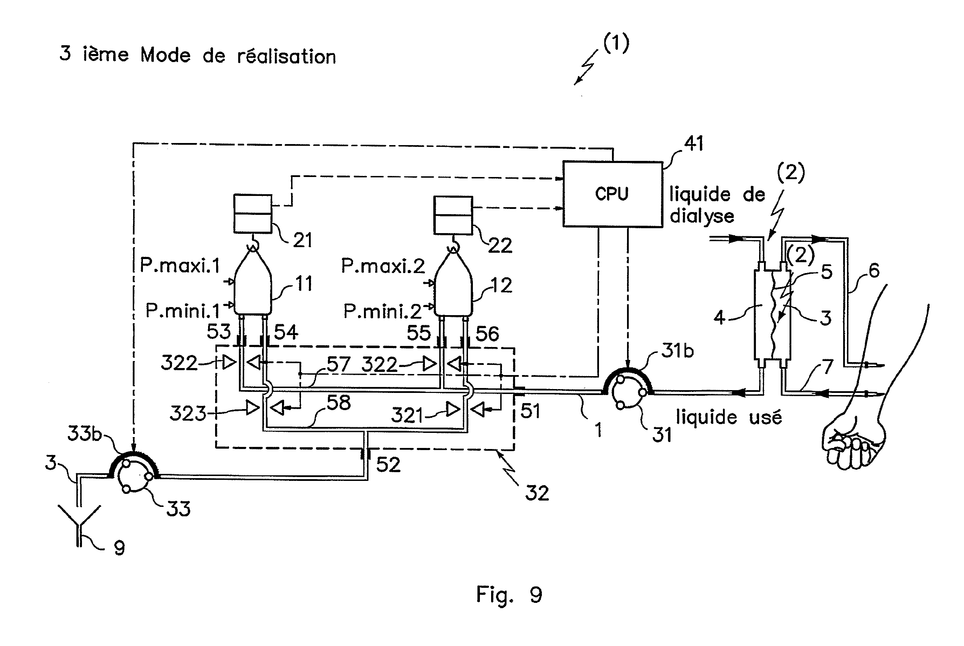

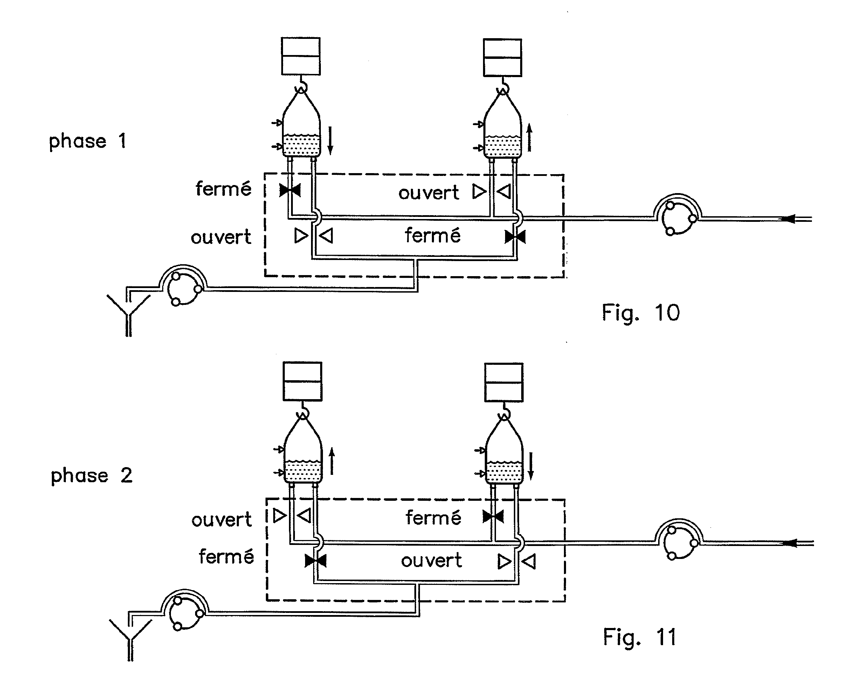

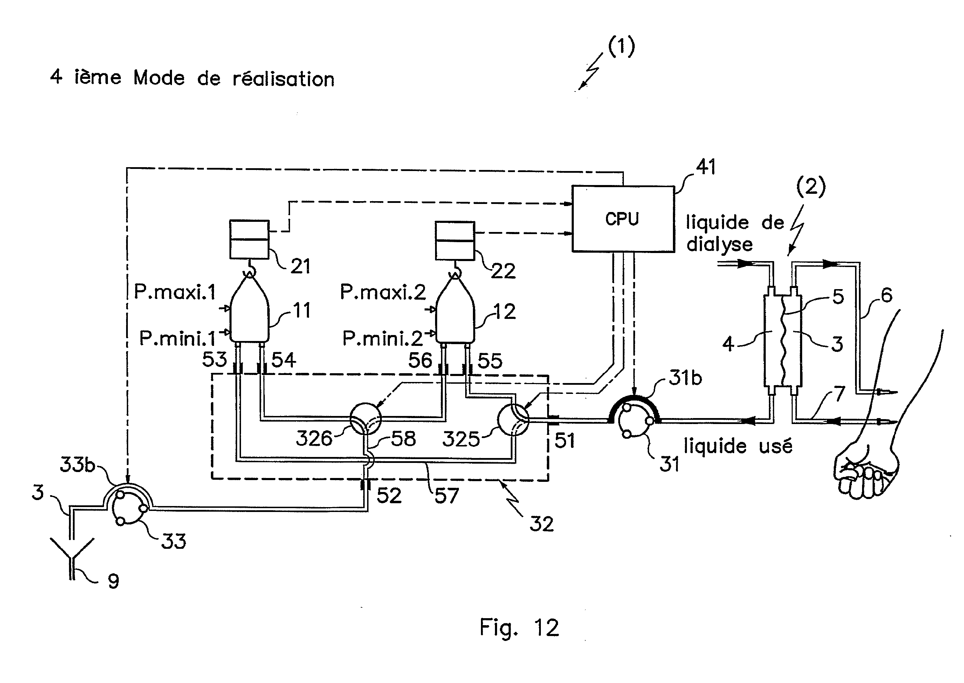

L'invention comporte également un troisième et un quatrième modes de réalisation illustrés respectivement dans les figures 9 et 12 et dont les deux phases de fonctionnement sont représentées respectivement dans les figures 10 et 11 ainsi que 13 et 14.

Dans ces deux modes, le deuxième organe de réglage 32 comporte un circuit hydraulique ayant six accès (51, 52, 53, 54, 55, 56) répartis de la façon suivante:

a) un premier accès en entrée 51 destiné à être en communication de fluide avec la portion d'entrée de la ligne de vidange 8 destinée à être reliée au filtre,
b) un deuxième accès en sortie 52 destiné à être en communication de fluide avec la portion de sortie de la ligne de vidange 8 destinée à être reliée à l'égout,
c) un troisième accès en entrée 53 et un quatrième accès en sortie 54 destinés chacun à être en communication de fluide avec le premier sac 11,

d) un cinquième accès en entrée 55 et un sixième accès en sortie 56 destinés chacun à être en communication de fluide avec le deuxième sac 12.

Description du troisième mode de réalisation :

Dans le troisième mode de réalisation, représenté en figure 9, le circuit hydraulique du deuxième organe de réglage 32 comporte deux parties.

La première partie comporte une première ligne 57 destinée à mettre en communication de fluide le premier accès en entrée 51 avec chacun des deux accès en sortie (54 et 56) destinés à communiquer avec chaque sac et deux clamps (322, 324) placés respectivement sur chaque portion de la première ligne 57 connectée auxdits deux accès en sortie (54 et 56).

La seconde partie comporte une deuxième ligne 58 destinée à mettre en communication de fluide le deuxième accès en sortie 52 avec chacun des deux accès en entrée (53 et 55) destinés à communiquer avec chaque sac, deux autres clamps (321, 323) placés respectivement sur chaque portion de la deuxième ligne 58 connectée auxdits deux accès en entrée (53 et 55).

Description du quatrième mode de réalisation :

Dans le quatrième mode de réalisation, représenté en figure 12, la structure du circuit hydraulique du deuxième organe de réglage 32 est différente, même si le fonctionnement sera le même.

En effet, le deuxième moyen de réglage 32 comporte deux parties.

La première partie comporte une première ligne 57 destinée à mettre en communication de fluide le premier accès en entrée 51 avec chacun des deux accès en sortie (53, 55) destinés à communiquer avec chaque sac et un premier clamp trois voies 325 pouvant adopter deux positions alternées. La première position est la mise en communication de fluide du premier accès en entrée 51 avec le troisième accès en sortie 53 au niveau du premier sac 11. La seconde position est la mise en communication de fluide du premier accès en entrée 51 avec le cinquième accès en sortie 55 au niveau du deuxième sac 12.

La seconde partie comporte une deuxième ligne 58 destinée à mettre en communication de fluide le deuxième accès en sortie 52 avec chacun des deux accès en entrée (54, 56) destinés à communiquer avec chaque sac et un deuxième clamp trois voies 326 pouvant adopter deux positions alternées suivantes. Une première position est la mise en communication de fluide du deuxième accès en sortie 52 avec le sixième accès en entrée 56, au niveau du deuxième sac 12. La seconde position est la mise en communication de fluide du deuxième accès en sortie 52 avec le quatrième accès en entrée 54, au niveau du premier sac 11.

Et, pour le troisième ou le quatrième mode de réalisation, le mode de fonctionnement alterné de charge et de décharge est identique. En effet, l'unité de contrôle 41 commande simultanément les clamps (321, 323, 323, 324, 325, 326) du deuxième organe de réglage 32 de sorte que deux phases sont alternées durant le fonctionnement.

En première phase, le deuxième sac 12 se charge de liquide tandis que le premier sac 11 se décharge vers l'égout 9.

En deuxième phase, le premier sac 11 se charge de liquide usé tandis que le deuxième sac 12 se décharge vers l'égout 9.

Il faut remarquer que pendant les deux phases, le troisième organe de réglage (33) assure un débit substantiellement continu, c'est-'à-dire que du liquide usé est continuellement envoyée vers l'égout.

L'invention concerne également une ligne jetable ou à usage unique (« disposable » en anglais) pour l'utilisation dans le dispositif selon l'invention.

Dans tout mode de réalisation, cette ligne jetable comprend au moins deux sacs et quatre parties de lignes dont:

- une première partie de ligne destinée à conduire du liquide depuis l'entrée de la ligne jetable (80) vers un des deux sacs (11,12);
- une deuxième partie de ligne destinée à conduire du liquide contenu dans ledit sac vers la sortie de la ligne jetable (80);
- une troisième partie de ligne destinée à conduire du liquide depuis l'entrée de la ligne jetable (80) vers l'autre sac (12, 11);
- une quatrième partie de ligne destinée à conduire du liquide stocké dans l'autre sac (12, 11) vers la sortie de la ligne jetable (80).

Pour les premier et deuxième mode de réalisation décrits, les première et troisième parties ont un tronçon commun reliant les deux sacs.

En effet, dans le premier et le deuxième mode de réalisation : la première partie de ligne est constituée par une partie du conduit 80 depuis l'entrée de la ligne 80 jusqu'au deuxième raccord 82 ou 821 et par le deuxième tronçon ou raccord 82. La deuxième partie de la ligne est constituée par le tronçon ou raccord 82 et par une partie du conduit 80 entre le raccord 821 ou 82 et la sortie de la ligne vers l'égout. La troisième partie de la ligne est constituée par une partie du conduit 80 depuis l'entrée de la ligne jusqu'au premier raccord 81 ou 811 et par le premier branchement ou raccord 81. La quatrième partie de la ligne est constituée par le premier raccord ou branchement 82 et par la partie du conduit 80 entre le premier raccord ou branchement 81 ou 811 et la sortie de la ligne.

Dans tout mode de réalisation, la ligne jetable comprend une ligne de vidange 80 destinée raccorder la sortie du filtre 2 à l'égout 9, deux sacs (11, 12) rattachés chacun à la ligne de vidange 8 et destinés à être accrochés à l'appareil de traitement 1, et au moins deux parties (31b, 33b) de la ligne de vidange 8 destinées à coopérer respectivement avec le premier organe de réglage 31 et le troisième organe de réglage 33.

Dans les premier et deuxième modes de réalisation, la ligne jetable comprend un conduit 80 , et au moins deux raccordements (81, 82) sur le conduit 80.

Dans le premier mode de réalisation, la ligne jetable comporte une autre partie 32b du conduit 80 placée entre les deux raccordements et destinée à coopérer avec le deuxième organe de réglage 32.

Dans le deuxième mode de réalisation, la ligne jetable comprend une quatrième partie (34b) placée sur le premier branchement (81) et destinée à coopérer avec le quatrième organe de réglage (34).

Dans le troisième mode de réalisation, la ligne jetable comprend une portion d'entrée de la ligne, une ligne de vidange 8 et une portion de sortie de la ligne.

La ligne de vidange 8 comporte une première canalisation de ligne 57 destiné à mettre en communication de fluide la portion d'entrée de la ligne de vidange et un accès à chaque sac, la première ligne étant en forme de T, et comporte une deuxième canalisation de ligne 58 destiné à mettre en communication de fluide la portion de sortie de la ligne de vidange et un second accès à chaque sac, la deuxième ligne étant en forme de T.

Dans le quatrième mode de réalisation, la ligne jetable comprend une portion d'entrée de la ligne, une ligne de vidange 8 et une portion de sortie de la ligne.

La ligne de vidange 8 comporte une première canalisation 57 destiné à mettre en communication de fluide la portion d'entrée de la ligne de vidange et un accès à chaque sac, la première canalisation comportant une vanne trois voies avec deux entrées et une sortie pour la connexion sélective de la sortie avec une des deux sorties,

La ligne de vidange 8 comporte également une deuxième canalisation 58 destiné à mettre en communication de fluide la portion de sortie de la ligné de vidange et un second accès à chaque sac, la deuxième canalisation comportant une vanne trois voies avec deux entrées et une sortie pour la connexion sélective de la sortie avec une des deux sorties.

Une telle ligne jetable peut être placée avant le début de la séance sur le dispositif de traitement extracorporel. A la fin de la séance, cette ligne sera déconnectée, jetée et remplacée par une nouvelle ligne pour la séance suivante.

Un procédé de vidange automatique d'une ligne de vidange, correspondant au dispositif selon l'invention sera discuté

Le procédé comporte deux phases alternées successives ayant les étapes suivantes : le passage en continu d'un liquide usé à travers une ligne de vidange en sortie d'un filtre, la première phase et la deuxième phase successive et alternée à la première phase liquide.

La première phase comporte la charge d'un premier conteneur (sac par exemple) par le 1 liquide usé et la décharge d'un second conteneur (sac par exemple), l'atteinte d'un premier poids seuil mesuré. La seconde phase comporte la décharge du premier conteneur de liquidé vers un égout et charge du second conteneur de liquide usé, et l'atteinte d'un second poids seuil mesuré.

En d'autres termes, le procédé de vidange automatique peut comporter deux phases alternées successives :

- la première phase comporte la charge d'un premier sac (11, 12) par un liquide usé et la décharge d'un second sac (12,11) de liquide usé vers un égout en sortie de la ligne de vidange, cette phase s'arrêtant dès l'atteinte par au moins un des deux sacs (11, 12) d'un premier poids seuil mesuré,
- la seconde phase comporte la décharge du premier sac de liquide (11, 12) vers un égout en sortie de la ligne de vidange et la charge du second sac (12, 11) de liquide usé, cette phase s'arrêtant dès l'atteinte d'un second poids seuil mesuré sur le même sac et/ou sur l'autre sac.

Les multiples avantages obtenus par l'invention sont les suivants :

- un contrôle du débit de liquide usé passant à travers la ligne de vidange est connu et contrôlé,
- la durée de séance avec le dispositif à vidange automatique est moindre à la durée d'une séance sans vidange automatique,
- le personnel soignant n'a plus à intervenir pour effectuer l'opération de changement de sac,
- le poids du liquide usé extrait du filtre est connu et maîtrisé
- les résultats, qualités du traitement utilisé selon l'état de l'art sont conservés,
- le niveau de sécurité assuré par le dispositif de traitement est conservé,
- l'équilibre hydrique est maintenu,
- le coût du traitement est diminué car deux sacs sont utilisés au lieu de plusieurs sacs remplacés successivement,
- le premier sac associé à son premier moyen de pesage gravimétrique et la portion de sortie de la ligne de vidange et l'égout peuvent être placés non pas sur le dispositif, mais placé dans un dispositif ou une pièce séparée du dispositif de traitement afin d'assurer une parfaite séparation entre le dispositif et le patient et la vidange : ceci renforce la sécurité du traitement.

**Revendications**

1. Dispositif de traitement de sang par circulation extracorporelle (1) comportant :

   - un filtre (2) ayant une chambre primaire (3) et une chambre secondaire (4) séparées par une membrane semi-perméable (5),
   - un circuit sang comportant une ligne artérielle (6) destinée à sortir du patient, la chambre primaire (3) du filtre et une ligne veineuse (7) destiné à retourner au patient,
   - un circuit dialysat comportant la chambre secondaire (4) du filtre et une ligne de vidange (8) configurée pour circuler la totalité du liquide usé sortant du filtre (2) vers un égout (9),
   - un premier sac (11) en communication de fluide avec la ligne de vidange (8),
   - un second sac (12) en communication de fluide avec la ligne de vidange (8),
   - au moins un premier moyen de pesage gravimétrique (21) associé au premier sac (11),
   - des moyens de réglage de débit de fluide (31,32,33,34) actifs sur la ligne de vidange (8), où les moyens de réglage comportent un premier organe de réglage (31) actif en amont des deux sacs (11, 12), un deuxième organe de réglage (32) actif entre les deux sacs (11, 12), et un troisième organe de réglage (33) actif en aval des deux sacs (11, 12),
   - une unité de contrôle (41) reliée au premier moyen de pesage gravimétrique (21) et aux moyens de réglage de débit de fluide (31,32,33,34),
   - où l'unité de contrôle (41) est capable de:

     i. recevoir les signaux de poids du premier moyen de pesage gravimétrique et,
     ii. commander les moyens de réglage de débit de fluide (31,32,33,34) pour charger de liquide un des sacs (11, 12) pendant que l'autre sac (12, 11) se décharge de liquide, et vice-versa, et
     iii. commander le premier organe de réglage (31) pour garantir la présence d'un débit substantiellement continu pendant le traitement.

2. Dispositif selon la revendication 1 **caractérisé en ce que** l'unité de contrôle (41) est capable de calculer, à partir des signaux de poids reçus, la quantité de liquide sortant du filtre et entrant dans la ligne de vidange (8).

3. Dispositif selon une des revendications 1 à 2, **caractérisé en ce que** la ligne de vidange (8) comprend :

   - un conduit (80) destiné à relier le filtre (2) à l'égout (9),
   - un premier branchement (81) reliant le premier sac (11) au conduit (80),
   - un deuxième branchement (82) reliant le second sac (12) au conduit (80),
   - le deuxième branchement (82) étant connecté au conduit (80) en amont du premier branchement (81),

et **en ce que** le premier organe de réglage (31) et le deuxième organe de réglage (32) sont actifs sur le conduit (80).

4. Dispositif selon la revendication 3 **caractérisé en ce que** chaque branchement (81, 82) comporte une ligne avec deux raccords terminaux respectifs (811, 812, 821, 822) et/ou chaque branchement (81, 82) comporte un raccord direct entre la ligne de vidange (80) et une ouverture d'un sac.

5. Dispositif selon l'une des revendications 3 à 4 **caractérisé en ce que** les premier (31) et troisième (33) organes de réglage sont des pompes péristaltiques, et le deuxième organe de réglage (32) est une vanne.

6. Dispositif selon la revendication 5 **caractérisé en ce que** le premier sac (11) est placé plus bas que le second sac (12) de sorte que le premier sac (11) se charge en priorité et le second sac (12) se décharge dans le premier sac (11) par gravité lorsque le deuxième organe de réglage (32) est ouvert.

7. Dispositif selon la revendication 5 ou 6 **caractérisé en ce que** l'unité de contrôle (41) est capable de commander les organes de réglage (31, 32, 33) selon les deux phases alternées suivantes :

   - en première phase : l'unité de contrôle (41) commande la fermeture du deuxième organe de réglage (32) et l'arrêt du troisième organe de réglage (33) pour la charge du second sac (12) et la décharge du premier sac (11) dans l'égout (9),
   - en seconde phase : l'unité de contrôle (41) commande l'ouverture du deuxième organe de réglage (32) et l'arrêt du troisième organe de réglage (33) pour la décharge du second sac (12) et la charge du premier sac (11).

8. Dispositif selon la revendication 4 **caractérisé en ce que** chaque branchement (81, 82) comporte une ligne avec deux raccords terminaux respectifs (811, 812, 821, 822), **en ce que** les moyens de réglage comportent un quatrième organe de réglage (34) actif sur le premier branchement (81) entre le raccord (811) et le premier sac.

9. Dispositif selon la revendication 8 **caractérisé en ce que** le premier et le quatrième organes de réglage (31, 34) sont des pompes péristaltiques et les deuxième et troisième organes de réglage (32,33) sont des vannes.

10. Dispositif selon la revendication 9 **caractérisé en ce**

**que** l'unité de contrôle (41) est capable de commander les moyens de réglage de débit (31, 32, 33, 34) selon les deux phases alternées suivantes:

   - en première phase : l'unité de contrôle (41) commande la fermeture du deuxième organe de réglage (32), l'ouverture du troisième organe de réglage (33), l'actionnement du quatrième organe de réglage (34) dans le sens sac-conduit,
   - en seconde phase : l'unité de contrôle (41) commande l'ouverture du deuxième organe de réglage (32), la fermeture du troisième organe de réglage (33), l'actionnement du quatrième organe de réglage (34) dans le sens conduit-sac.

11. Dispositif selon une des revendications 1 à 2 **caractérisé en ce que** le deuxième organe de réglage (32) comporte un circuit hydraulique ayant six accès (51, 52, 53, 54, 55, 56) répartis de la façon suivante:

   un premier accès en entrée (51) destiné à être en communication de fluide avec la portion d'entrée de la ligne de vidange (8) destinée à être reliée au filtre (2),
   un deuxième accès en sortie (52) destiné à être en communication de fluide avec la portion de sortie de la ligne de vidange (8) destinée à être reliée à l'égout (9),
   un troisième accès en entrée (53) et un quatrième accès en sortie (54) destinés chacun à être en communication de fluide avec le premier sac (11),
   un cinquième accès en entrée (55) et un sixième accès en sortie (56) destinés chacun à être en communication de fluide avec le deuxième sac (12).

12. Dispositif selon la revendication 11 **caractérisé en ce que** le circuit hydraulique du deuxième organe de réglage (32) comporte:

   - une première ligne (57) destinée à mettre en communication de fluide le premier accès en entrée (51) avec chacun des deux accès en sortie (54 et 56) destinés à communiquer avec chaque sac,
   - deux clamps (322, 324) placés respectivement sur chaque portion de la première ligne (57) connectée auxdits deux accès en sortie (54 et 56),

et

   - une deuxième ligne (58) destinée à mettre en communication de fluide le deuxième accès en sortie (52) avec chacun des deux accès en entrée (53 et 55) destinés à communiquer avec chaque sac,
   - deux autres clamps (321, 323) placés respectivement sur chaque portion de la deuxième ligne (58) connectée auxdits deux accès en entrée (53 et 55);

ou **en ce que**
le circuit hydraulique du deuxième organe de réglage (32) comporte:

- une première ligne (57) destinée à mettre en communication de fluide le premier accès en entrée (51) avec chacun des deux accès en sortie (53, 55) destinés à communiquer avec chaque sac et une première vanne trois voies (325) pouvant adopter les deux positions alternées suivantes:

o première position : mise en communication de fluide du premier accès en entrée (51) avec le troisième accès en sortie (53) au niveau du premier sac (11),
o seconde position: mise en communication de fluide du premier accès en entrée (51) avec le cinquième accès en sortie (55) au niveau du deuxième sac (12),

- une deuxième ligne (58) destinée à mettre en communication de fluide le deuxième accès en sortie (52) avec chacun des deux accès en entrée (54, 56) destinés à communiquer avec chaque sac et une deuxième vanne trois voies (326) pouvant adopter les deux positions alternées suivantes correspondantes:

◦ première position: mise en communication de fluide du deuxième accès en sortie (52) avec le sixième accès en entrée (56), au niveau du deuxième sac (12),
◦ seconde position: mise en communication de fluide du deuxième accès en sortie (52) avec le quatrième accès en entrée (54), au niveau du premier sac (11).

13. Dispositif selon la revendication 12 caractérisé en ce l'unité de contrôle (41) commande simultanément les clamps (321, 323, 323, 324, 325, 326) du deuxième organe de réglage (32) de sorte que les deux phases suivantes sont alternées durant le fonctionnement:

- en première phase: le deuxième sac (12) se charge de liquide tandis que le premier sac (11) se décharge vers l'égout (9),
- en deuxième phase: le premier sac (11) se charge de liquide usé tandis que le deuxième sac (12) se décharge vers l'égout (9),

et en ce que le troisième organe de réglage (33) assure un débit substantiellement continu.

14. Dispositif selon une des revendications précédentes **caractérisé en ce qu'**il comporte un second moyen de pesage gravimétrique (22) associé au second sac (12) et relié à l'unité de contrôle (41),
**en ce que** l'unité de contrôle (41) est capable de calculer la quantité de fluide sortant du filtre (2) et entrant dans la ligne de vidange (8) à partir des signaux reçus du premier moyen de pesage gravimétrique (21) et/ou du second moyen de pesage gravimétrique (22), et
en ce l'unité de contrôle (41) est capable de :

- recevoir l'information de poids du premier moyen de pesage gravimétrique (21) et/ou du second moyen de pesage gravimétrique (22),
- calculer le débit réel de fluide sortant du filtre (2) et le comparer à un débit souhaité,
- contrôler le débit réel de fluide par les moyens de réglage pour approcher le débit souhaité de fluide sortant du filtre (2).

15. Dispositif selon une des revendications précédentes caractérisé en ce l'unité de contrôle (41) est capable de:

- recevoir l'information de poids du premier moyen de pesage gravimétrique (21) et/ou du second moyen de pesage gravimétrique (22),
- déterminer indépendamment l'état de remplissage de chaque sac,
- commander, à partir de l'état de remplissage de chaque sac, une procédure de charge et décharge alternée et successive des sacs.

et/ou
en ce l'unité de contrôle (41) est capable de:

- recevoir l'information de poids du premier moyen de pesage gravimétrique (21) et/ou du second moyen de pesage gravimétrique (22),
- détecter des valeurs de seuil maximal et seuil minimal pour chacun des sacs $P_{1mini}$, $P_{1maxi}$, $P_{2mini}$, $P_{2maxi}$,
- commander à partir de valeurs de seuil une procédure de charge et décharge des sacs selon les étapes suivantes :

◦ charge d'un sac et décharge de l'autre sac,
◦ détection d'un seuil limite,
◦ décharge d'un sac et charge de l'autre sac,
◦ détection d'un autre seuil limite.

**Patentansprüche**

1. Vorrichtung zur Blutbehandlung durch extrakorporale Zirkulation (1) umfassend:

- einen Filter (2) mit einer Hauptkammer (3) und einer Nebenkammer (4), die durch eine semipermeable Membran (5) voneinander getrennt sind,

- einen Blutkreislauf umfassend eine arterielle Leitung (6) vorgesehen für den Ausgang vom Patienten, die Hauptkammer (3) des Filters und eine venöse Leitung (7) vorgesehen für den Rücklauf zum Patienten,

- einen Dialysatkreislauf umfassend die Nebenkammer (4) des Filters und eine Entleerungsleitung (8), die konfiguriert ist zum Zirkulieren der gesamten, aus dem Filter (2) auslaufenden gebrauchten Flüssigkeit zu einem Ablauf (9),

- einen ersten Beutel (11) in Fluidkommunikation mit der Entleerungsleitung (8),

- einen zweiten Beutel (12) in Fluidkommunikation mit der Entleerungsleitung (8),

- mindestens ein erstes Mittel zum gravimetrischen Wiegen (21), das dem ersten Beutel (11) zugeordnet ist,

- Mittel zur Einstellung des Fluiddurchsatzes (31, 32, 33, 34), die auf die Entleerungsleitung (8) wirken, wobei die Einstellungsmittel ein erstes Einstellungselement (31), das aufwärts von den beiden Beuteln (11, 12) wirkt, ein zweites Einstellungselement (32), das zwischen den beiden Beuteln (11, 12) wirkt, und ein drittes Einstellungselement (33), das abwärts von den beiden Beuteln (11, 12) wirkt, umfassen,

- eine Steuereinheit (41), die mit dem ersten Mittel zum gravimetrischen Wiegen (21) und mit den Mitteln zur Einstellung des Fluiddurchsatzes (31, 32, 33, 34) verbunden ist,

- wobei die Steuereinheit (41):

    i. die Gewichtssignale vom ersten Mittel zum gravimetrischen Wiegen empfangen kann, und

    ii. die Mittel zur Einstellung des Fluiddurchsatzes (31, 32, 33, 34) so steuern kann, einen der Beutel (11, 12) mit Flüssigkeit zu füllen, während der andere Beutel (12, 11) von der Flüssigkeit entleert wird, und umgekehrt, und

    iii. das erste Einstellungsmittel (31) so steuern kann, die Anwesenheit eines im Wesentlichen kontinuierlichen Durchsatzes während der Behandlung sicherzustellen.

2.  Vorrichtung nach Anspruch 1, **dadurch gekennzeichnet, dass** die Steuereinheit (41) ausgehend von den empfangenen Gewichtssignalen die Flüssigkeitsmenge berechnen kann, die aus dem Filter ausläuft und in die Entleerungsleitung (8) gelangt.

3.  Vorrichtung nach einem der Ansprüche 1 bis 2, **dadurch gekennzeichnet, dass** die Entleerungsleitung (8) umfasst:

    - eine Rohrleitung (80) zur Verbindung des Filters (2) mit dem Ablauf (9),

    - eine erste Verzweigung (81), die den ersten Beutel (11) mit der Rohrleitung (80) verbindet,

    - eine zweite Verzweigung (82), die den zweiten Beutel (12) mit der Rohrleitung (80) verbindet,

    - wobei die zweite Verzweigung (82) mit der Rohrleitung (80) aufwärts von der ersten Verzweigung (81) verbunden ist,

und dass das erste Einstellungselement (31) und das zweite Einstellungselement (32) auf die Rohrleitung (80) wirken.

4.  Vorrichtung nach Anspruch 3, **dadurch gekennzeichnet, dass** jede Verzweigung (81, 82) eine Leitung mit zwei jeweiligen Endanschlüssen (811, 812, 821, 822) umfasst und/oder jede Verzweigung (81, 82) einen unmittelbaren Anschluss zwischen der Entleerungsleitung (80) und einer Öffnung eines Beutels umfasst.

5.  Vorrichtung nach einem der Ansprüche 3 bis 4, **dadurch gekennzeichnet, dass** die ersten (31) und dritten (33) Einstellungselemente peristaltische Pumpen sind und das zweite Einstellungselement (32) ein Ventil ist.

6.  Vorrichtung nach Anspruch 5, **dadurch gekennzeichnet, dass** der erste Beutel (11) niedriger als der zweite Beutel (12) angeordnet ist, so dass zuerst der erste Beutel (11) gefüllt wird und dann der zweite Beutel (12) in den ersten Beutel (11) durch Schwerkraft entleert wird, wenn das zweite Einstellungselement (32) offen ist.

7.  Vorrichtung nach Anspruch 5 oder 6, **dadurch gekennzeichnet, dass** die Steuereinheit (41) die Einstellungselemente (31, 32, 33) nach den folgenden zwei alternierenden Schritten steuern kann:

    - als erster Schritt: Die Steuereinheit (41) steuert das Schließen des zweiten Einstellungselementes (32) und das Halten des dritten Einstellungselementes (33) zum Befüllen des zweiten Beutels (12) und zum Entleeren des ersten Beutels (11) in den Ablauf (9),

    - als zweiter Schritt: Die Steuereinheit (41) steuert das Öffnen des zweiten Einstellungselementes (32) und das Halten des dritten Einstellungselementes (33) zum Entleeren des zweiten Beutels (12) und zum Befüllen des ersten Beutels (11).

8.  Vorrichtung nach Anspruch 4, **dadurch gekennzeichnet, dass** jede Verzweigung (81, 82) eine Leitung mit zwei jeweiligen Endanschlüssen (811, 812, 821, 822) umfasst, dass die Einstellungsmittel ein viertes Einstellungselement (34) umfassen, das an der ersten Verzweigung (81) zwischen dem Anschluss und dem ersten Beutel wirkt.

9. Vorrichtung nach Anspruch 8, **dadurch gekennzeichnet, dass** die ersten und vierten Einstellungselemente (31, 34) peristaltische Pumpen sind und die zweiten und dritten Einstellungselemente (32, 33) Ventile sind.

10. Vorrichtung nach Anspruch 9, **dadurch gekennzeichnet, dass** die Steuereinheit (41) die Mittel zur Einstellung des Durchsatzes (31, 32, 33, 34) nach den folgenden zwei alternierenden Schritten steuern kann:

   - als erster Schritt: Die Steuereinheit (41) steuert das Schließen des zweiten Einstellungselementes (32), das Öffnen des dritten Einstellungselementes (33) und die Betätigung des vierten Einstellungselementes (34) in der Richtung Beutel-Rohrleitung,
   - als zweiter Schritt: Die Steuereinheit (41) steuert Öffnen des zweiten Einstellungselementes (32), das Schließen des dritten Einstellungselementes (33) und die Betätigung des vierten Einstellungselementes (34) in der Richtung Rohrleitung-Beutel.

11. Vorrichtung nach einem der Ansprüche 1 bis 2, **dadurch gekennzeichnet, dass** das zweite Einstellungselement (32) einen Hydraulikkreislauf mit sechs Zugängen (51, 52, 53, 54, 55, 56) umfasst, die wie folgt verteilt sind:

   einen ersten Eingangszugang (51) zur Fluidkommunikation mit dem Eingangsabschnitt der Entleerungsleitung (8) zur Verbindung mit dem Filter (2),
   einen zweiten Ausgangszugang (52) zur Fluidkommunikation mit dem Ausgangsabschnitt der Entleerungsleitung (8) zur Verbindung mit dem Ablauf (9),
   einen dritten Eingangszugang (53) und einen vierten Ausgangszugang (54), jeder zur Fluidkommunikation mit dem ersten Beutel (11),
   einen fünften Eingangszugang (55) und einen sechsten Ausgangszugang (56), jeder zur Fluidkommunikation mit dem zweiten Beutel (12).

12. Vorrichtung nach Anspruch 11, **dadurch gekennzeichnet, dass** der Hydraulikkreislauf des zweiten Einstellungselementes (32) umfasst:

   - eine erste Leitung (57) zum Setzen des ersten Eingangszugangs (51) in Fluidkommunikation mit jedem der beiden Ausgangszugänge (54 und 56) zur Kommunikation mit jedem Beutel,
   - zwei Klemmen (322, 324), die jeweils auf jedem Abschnitt der ersten Leitung (57) angeordnet sind, die mit den beiden Ausgangszugängen (54 und 56) verbunden ist,

und

   - eine zweite Leitung (58) zum Setzen des zweiten Ausgangszugangs (52) in Fluidkommunikation mit jedem der beiden Eingangszugänge (53 und 55) zur Kommunikation mit jedem Beutel,
   - zwei weitere Klemmen (321, 323), die jeweils auf jedem Abschnitt der zweiten Leitung (58) angeordnet sind, die mit den beiden Eingangszugängen (53 und 55) verbunden ist;

oder dass
der Hydraulikkreislauf des zweiten Einstellungselementes (32) folgendes umfasst:

   - eine erste Leitung (57) zum Setzen des ersten Eingangszugangs (51) in Fluidkommunikation mit jedem der beiden Ausgangszugänge (53, 55) zur Kommunikation mit jedem Beutel, und ein erstes Dreiwegeventil (325), das die folgenden zwei alternierenden Stellungen annehmen kann:

      ◦ erste Stellung: Setzen des ersten Eingangszuganges (51) in Fluidkommunikation mit dem dritten Ausgangszugang (53) am ersten Beutel (11),
      ◦ zweite Stellung: Setzen des ersten Eingangszuganges (51) in Fluidkommunikation mit dem fünften Ausgangszugang (55) am zweiten Beutel (12),

   - eine zweite Leitung (58) zum Setzen des zweiten Ausgangszugangs (52) in Fluidkommunikation mit jedem der beiden Eingangszugänge (54, 56) zur Kommunikation mit jedem Beutel, und ein zweites Dreiwegeventil (326), das die folgenden entsprechenden zwei alternierenden Stellungen annehmen kann:

      ◦ erste Stellung: Setzen des zweiten Ausgangszuganges (52) in Fluidkommunikation mit dem sechsten Eingangszugang (56) am zweiten Beutel (12),
      ◦ zweite Stellung: Setzen des zweiten Ausgangszuganges (52) in Fluidkommunikation mit dem vierten Eingangszugang (54) am ersten Beutel (11).

13. Vorrichtung nach Anspruch 12, **dadurch gekennzeichnet, dass** die Steuereinheit (41) die Klemmen (321, 323, 323, 324, 325, 326) des zweiten Einstellungselementes (32) gleichzeitig so steuert, dass die folgenden zwei Schritte sich während des Betriebes alternieren:

- als erster Schritt: Der zweite Beutel (12) wird mit Flüssigkeit gefüllt, während der erste Beutel (11) in den Ablauf (9) entleert wird,
- als zweiter Schritt: Der erste Beutel (11) wird mit gebrauchter Flüssigkeit gefüllt, während der zweite Beutel (12) in den Ablauf (9) entleert wird,

und dass das dritte Einstellungselement (33) einen im Wesentlichen kontinuierlichen Durchsatz sicherstellt.

14. Vorrichtung nach einem der vorherigen Ansprüche, **dadurch gekennzeichnet, dass** sie ein zweites Mittel zum gravimetrischen Wiegen (22) umfasst, das dem zweiten Beutel (12) zugeordnet ist und mit der Steuereinheit (41) verbunden ist, dass die Steuereinheit (41) ausgehend von den vom ersten Mittel zum gravimetrischen Wiegen (21) und/oder vom zweiten Mittel zum gravimetrischen Wiegen (22) empfangenen Gewichtssignalen die Flüssigkeitsmenge berechnen kann, die aus dem Filter (2) ausläuft und in die Entleerungsleitung (8) gelangt, und dass die Steuereinheit (41):

- die Gewichtsinformation vom ersten Mittel zum gravimetrischen Wiegen (21) und/oder vom zweiten Mittel zum gravimetrischen Wiegen (22) empfangen kann,
- den effektiven Durchsatz des aus dem Filter (2) auslaufenden Fluids berechnen und mit einem gewünschten Durchsatz vergleichen kann,
- den effektiven Fluiddurchsatz durch die Einstellungsmittel steuern kann, um dem gewünschten Durchsatz des aus dem Filter (2) auslaufenden Fluids näher zu kommen.

15. Vorrichtung nach einem der vorherigen Ansprüche, **dadurch gekennzeichnet, dass** die Steuereinheit (41) :

- die Gewichtsinformation vom ersten Mittel zum gravimetrischen Wiegen (21) und/oder vom zweiten Mittel zum gravimetrischen Wiegen (22) empfangen kann,
- unabhängig den Füllzustand jedes Beutels bestimmen kann,
- ausgehend vom Füllzustand jedes Beutels eine alternierende und aufeinanderfolgende Füll- und Entleerungsprozedur steuern kann,

und/oder
dass die Steuereinheit (41):

- die Gewichtsinformation vom ersten Mittel zum gravimetrischen Wiegen (21) und/oder vom zweiten Mittel zum gravimetrischen Wiegen (22) empfangen kann,
- Maximalschwellenwerte und Minimalschwellenwerte für jeden Beutel $P_{1mini}$, $P_{1maxi}$, $P_{2mini}$, $P_{2maxi}$ erfassen kann,
- ausgehend von den Schwellenwerten eine Füll- und Entleerungsprozedur nach den folgenden Schritten steuern kann:

  ◦ Füllen eines Beutels und Entleeren des anderen Beutels,
  ◦ Erfassen einer Grenzschwelle,
  ◦ Entleeren eines Beutels und Füllen des anderen Beutels,
  ◦ Erfassen einer anderen Grenzschwelle.

**Claims**

1. A device for blood treatment by means of extracorporeal circulation (1) comprising:

   - a filter (2) having a primary compartment (3) and a secondary compartment (4) separated by a semipermeable membrane (5),
   - a blood circuit comprising an arterial line (6) designed to withdraw from the patient, the primary compartment (3) of the filter and a venous line (7) designed to return to the patient,
   - a dialysate circuit comprising the secondary compartment (4) of the filter and a draining line (8) configured to let the whole of the used liquid getting out of the filter (2) to go towards a drain (9),
   - a first bag (11) in fluid communication with the draining line (8),
   - a second bag (12) in fluid communication with the draining line (8),
   - at least a first gravimetric weighing means (21) associated with the first bag (11),
   - fluid flow rate adjusting means (31, 32, 33, 34) acting upon the draining line (8), wherein the adjusting means comprise a first adjusting member (31) acting upstream from the two bags (11, 12), a second adjusting member (32) acting between the two bags (11, 12), and a third adjusting member (33) acting downstream from the two bags (11, 12),
   - a control unit (41) connected to the first gravimetric weighing means (21) and to the fluid flow rate adjustment means (31, 32, 33, 34),
   - wherein the control unit (41) is capable to:

     i. receive the weight signals from the first gravimetric weighing means, and
     ii. piloting the fluid flow rate adjustment means (31, 32, 33, 34) so as to fill one of the bags (11, 12) with liquid while the other bag (12, 11) is emptied of the liquid, and vice versa, and

iii. piloting the first adjusting member (31) so as to ensure the presence of a substantially continuous flow rate during treatment.

2. The device according to claim 1, **characterized in that** the control unit (41) is capable to calculate, starting from the received weight signals, the amount of liquid withdrawn from the filter and entering the draining line (8).

3. The device according to one of the claims 1 to 2, **characterized in that** the draining line (8) comprises:

- a tube (80) designed to connect the filter (2) to the drain (9),
- a first branch (81) connecting the first bag (11) to the tube (80),
- a second branch (82) connecting the second bag (12) to the tube (80),
- the second bag (82) being connected to the tube (80) upstream from the first branch (81), and **in that** the first adjustment member (31) and the second adjusting member (32) act upon the tube (80).

4. The device according to claim 3, **characterized in that** each branch (81, 82) comprises a line with two respective end connections (811, 812, 821, 822) and/or each branch (81, 82) comprises a direct connection between the draining line (80) and an opening of a bag.

5. The device according to one of the claims 3 to 4, **characterized in that** the first (31) and third (33) adjusting members are peristaltic pumps, and the second adjusting member (32) is a valve.

6. The device according to claim 5, **characterized in that** the first bag (11) is placed in a lower position than the second bag (12) so that the first bag (11) is filled first and the second bag (12) is emptied into the first bag (11) by gravity when the second adjusting member (32) is open.

7. The device according to claim 5 or 6, **characterized in that** the control unit (41) is capable to pilot the adjusting members (31, 32, 33) according to the following two alternating steps:

- as first step: the control unit (41) pilots the closing of the second adjusting member (32) and the stopping of the third adjusting member (33) for filling the second bag (12) and emptying the first bag (11) into the drain (9),
- as second step: the control unit (41) pilots the opening of the second adjusting member (32) and the stopping of the third adjusting member (33) for emptying the second bag (12) and filling the first bag (11).

8. The device according to claim 4, **characterized in that** each branch (81, 82) comprises a line with two respective end connections (811, 812, 821, 822), **in that** the adjusting means comprise a fourth adjusting member (34) acting upon the first branch (81) between the connection (811) and the first bag.

9. The device according to claim 8, **characterized in that** the first and fourth adjusting members (31, 34) are peristaltic pumps, and the second and third adjusting members (32, 33) are valves.

10. The device according to claim 9, **characterized in that** the control unit (41) is capable to pilot the flow rate adjusting means (31, 32, 33, 34) according to the following two alternating steps:

- as first step: the control unit (41) pilots the closing of the second adjusting member (32), the opening of the third adjusting member (33) and the actuation of the fourth adjusting member (34) in the direction bag-tube,
- as second step: the control unit (41) pilots the opening of the second adjusting member (32), the closing of the third adjusting member (33) and the actuation of the fourth adjusting member (34) in the direction tube-bag.

11. The device according to one of the claims 1 to 2, **characterized in that** the second adjusting member (32) comprises a hydraulic circuit having six accesses (51, 52, 53, 54, 55, 56) distributed as follows:

a first inlet access (51) designed to be in fluid communication with the inlet portion of the draining line (8) designed to be connected to the filter (2),
a second outlet access (52) designed to be in fluid communication with the outlet portion of the draining line (8) designed to be connected to the drain (9),
a third inlet access (53) and a fourth outlet access (54) each designed to be in fluid communication with the first bag (11),
a fifth inlet access (55) and a sixth outlet access (56) each designed to be in fluid communication with the second bag (12).

12. The device according to claim 11, **characterized in that** the hydraulic circuit of the second adjusting member (32) comprises:

- a first line (57) designed to set in fluid communication the first inlet access (51) with each one of the two outlet accesses (54 and 56) designed to communicate with each bag,
- two clamps (322, 324) placed on each portion of the first line (57) connected to said two outlet accesses (54 and 56), respectively,

and

- a second line (58) designed to set in fluid communication the second outlet access (52) with each one of the two inlet accesses (53 and 55) designed to communicate with each bag,
- two further clamps (321, 323) placed on each portion of the second line (58) connected to said two inlet accesses (53 and 55), respectively;

or **in that**
the hydraulic circuit of the second adjusting member (32) comprises:

- a first line (57) designed to set in fluid communication the first inlet access (51) with each one of the two outlet accesses (53, 55) designed to communicate with each bag, and a first three-way valve (325) being able to take the following two alternating positions:

  ∘ first position: setting in fluid communication the first inlet access (51) with the third outlet access (53) on the first bag (11),
  ∘ second position: setting in fluid communication the first inlet access (51) with the fifth outlet access (55) on the second bag (12),

- a second line (58) designed to set in fluid communication the second outlet access (52) with each one of the two inlet accesses (54, 56) designed to communicate with each bag, and a second three-way valve (326) being able to take the following two corresponding alternating positions:

  ∘ first position: setting in fluid communication the second outlet access (52) with the sixth inlet access (56) on the second bag (12),
  ∘ second position: setting in fluid communication the second outlet access (52) with the fourth inlet access (54) on the first bag (11).

13. The device according to claim 12, **characterized in that** the control unit (41) simultaneously pilots the clamps (321, 323, 323, 324, 325, 326) of the second adjusting member (32) so that the two following steps alternate during operation:

- as first step: the second bag (12) is filled with liquid while the first bag (11) is emptied towards the drain (9),
- as second step: the first bag (11) is filled with used liquid while the second bag (12) is emptied towards the drain (9),

and **in that** the third adjusting member (33) ensures a substantially continuous flow rate.

14. The device according to one of the preceding claims, **characterized in that** it comprises a second gravimetric weighing means (22) associated to the second bag (12) and connected to the control unit (41), and **in that** the control unit (41) is capable to calculate the amount of fluid getting out of the filter (2) and entering the draining line (8) starting from the signals received from the first gravimetric weighing means (21) and/or from the second gravimetric weighing means (22), and
**in that** the control unit (41) is capable to:

- receive the weight information from the first gravimetric weighing means (21) and/or from the second gravimetric weighing means (22),
- calculate the real fluid flow rate getting out of the filter (2) and comparing it with a desired flow rate,
- control the real fluid flow rate through the adjusting means so as to get near the desired fluid flow rate getting out of the filter (2).

15. The device according to one of the preceding claims, **characterized in that** the control unit (41) is capable to:

- receive the weight information from the first gravimetric weighing means (21) and/or from the second gravimetric weighing means (22),
- separately determine the filling level of each bag,
- pilot, starting from the filling level of each bag, an alternating and sequential filling and emptying procedure for the bags,

and/or
**in that** the control unit (41) is capable to:

- receive the weight information from the first gravimetric weighing means (21) and/or from the second gravimetric weighing means (22),
- detect maximum and minimum threshold values for each one of the bags $P_{1mini}$, $P_{1maxi}$, $P_{2mini}$, $P_{2maxi}$,
- pilot, starting from the threshold values, a filling and emptying procedure for the bags according to the following steps:

◦ filling a bag and emptying the other bag,
◦ detecting a limit threshold,
◦ emptying a bag and filling the other bag,
◦ detecting another limit threshold.

Etat de l'art

Fig. 1

1er Mode de réalisation (1)

CPU 41

P.maxi.2 22
P.mini.2 12
822
82

P.maxi.1
P.mini.1 21
11
812
81
811

liquide de dialyse (2)
5
6
4 3
7

33b 31b
33 80 32 32b 821 31 liquide usé
9 (8)

Fig. 2

EP 2 338 542 B1

19

phase 1

fermé

Fig. 3

phase 2

ouvert

Fig. 4

Fig. 5

2eme Mode de réalisation

Fig. 6

phase 1

ouvert       fermé

**Fig. 7**

phase 2

fermé       ouvert

**Fig. 8**

3 ième Mode de réalisation

Fig. 9

EP 2 338 542 B1

phase 1

ouvert

fermé

ouvert

fermé

Fig. 10

phase 2

fermé

ouvert

ouvert

fermé

Fig. 11

4 ième Mode de réalisation

Fig. 12

phase 1

Fig. 13

phase 2

Fig. 14

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0796997 A
- US 4859319 A
- US 50430745 B